# EUROPÄISCHE PATENTSCHRIFT

(11) **EP 0 406 656 B1**
(45) Veröffentlichungstag und Bekanntmachung des Hinweises auf die Patenterteilung: **11.01.1995**
(21) Anmeldenummer: 90111996.6
(22) Anmeldetag: 25.06.1990
(51) Int. Cl.: C07D 405/04, A61K 31/35, A61K 31/44

(54) **Chromanderivate**
Chroman derivatives
Dérivés de chromane

(30) Priorität: 07.07.1989 DE 3922392
(43) Veröffentlichungstag der Anmeldung: 09.01.1991
(73) Patentinhaber: MERCK PATENT GmbH, D-64271 Darmstadt (DE)
(72) Erfinder: Gericke, Rolf, Dr., D-6104 Seeheim (DE); Baumgarth, Manfred, Dr., D-6100 Darmstadt (DE); Lues, Ingeborg, Dr., D-6100 Darmstadt (DE); Bergmann, Rolf, Dr., D-6101 Reichelsheim (DE); De Peyer, Jacques, Dr., D-3007 Bern (DE)

(56) Entgegenhaltungen:
- EP-A- 0 218 373
- EP-A- 0 273 262
- EP-A- 0 298 452

## Beschreibung

Die Erfindung betrifft neue Chromanderivate der Formel I 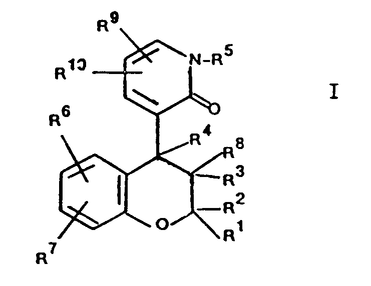
worin
- R¹ und R²: jeweils A,
- R⁸: H,
- R³: OH,
- R⁴: H,
- R³ und R⁴: zusammen auch eine Bindung,
- R⁵: A oder einen unsubstituierten oder ein-, zwei- oder dreifach durch A und/oder OA substituierten Benzylrest,
- R⁶ und R⁷: jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂ HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
- R⁹ und R¹⁰: jeweils H, A, F, Cl, Br, J, OH, OA, OAc, SH, NO₂, NH₂, NHAc, COOH oder COOA,
- A: Alkyl mit 1-6 C-Atomen,
- -alkyl: Alkylen mit 1-6 C-Atomen und
- Ac: Alkanoyl mit 1-8 C-Atomen oder Aroyl mit 7-11 C-Atomen
bedeuten,
sowie deren Salze.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre physiologisch unbedenklichen Salze bei guter Verträglichkeit wertvolle pharmakologische Eigenschaften besitzen. So zeigen sie Wirkungen auf das cardiovaskuläre System, wobei in der Regel bei niedrigeren Dosen ein selektiver Angriff am Coronarsystem, bei höheren ein blutdrucksenkender Effekt beobachtet werden kann. Am Coronarsystem treten z.B. Widerstandsabnahme und Flußzunahme auf, wobei der Einfluß auf die Herzfrequenz gering bleibt. Weiterhin zeigen die Verbindungen eine relaxierende Wirkung auf verschiedene glattmuskulare Organe (Gastrointestinaltrakt, Respirationssystem und Uterus). Die Wirkungen der Verbindungen können mit Hilfe an sich bekannter Methoden ermittelt werden, wie sie z.B. in der EP-A1-76075, der EP-A1-173848 oder der AU-A-45547/85 (Derwent Farmdoc Nr. 86081769) sowie von K.S. Meesmann et al., Arzneimittelforschung 25 (11), 1975, 1770-1776, angegeben sind. Als Versuchstiere eignen sich z.B. Mäuse, Ratten, Meerschweinchen, Hunde, Katzen, Affen oder Schweine.

Die Verbindungen können daher als Arzneimittelwirkstoffe in der Human- und Veterinärmedizin verwendet werden. Ferner können sie als Zwischenprodukte zur Herstellung weiterer Arzneimittelwirkstoffe verwendet werden.

In den angegebenen Formeln bedeutet A eine vorzugsweise unverzweigte Alkylgruppe mit 1-6, bevorzugt 1-4, insbesondere 1, 2 oder 3 C-Atomen, im einzelnen vorzugsweise Methyl, ferner bevorzugt Ethyl, Propyl, Isopropyl, Butyl, Isobutyl, weiterhin bevorzugt sek.-Butyl, tert.-Butyl, Pentyl, Isopentyl (3-Methylbutyl), Hexyl oder Isohexyl (4-Methylpentyl).

Falls R¹ und R² zusammen Alkylen bedeuten, so ist die Alkylengruppe vorzugsweise unverzweigt, im einzelnen bevorzugt -(CH₂)ₙ-, wobei n 3, 4, 5 oder 6 bedeutet.

Die Gruppe "-alkyl" steht vorzugsweise für -CH₂- oder -CH₂CH₂-.

Ac ist vorzugsweise Alkanoyl mit 1-6, insbesondere 1 ,2, 3 oder 4 C-Atomen, im einzelnen bevorzugt Formyl oder Acetyl, weiterhin bevorzugt Propionyl, Butyryl, Isobutyryl, Pentanoyl oder Hexanoyl, ferner bevorzugt Benzoyl, o-, m- oder p-Toluyl, 1- oder 2-Naphthoyl.

R¹ und R² sind vorzugsweise jeweils Alkyl, insbesondere jeweils Methyl oder Ethyl, bevorzugt jeweils Methyl; weiterhin sind R¹ und R² zusammen bevorzugt -(CH₂)₄-oder -(CH₂)₅-.

Falls R⁴ H bedeutet, ist R³ bevorzugt OH, ferner bevorzugt O-CHO oder O-COCH₃.

R⁵ ist A (insbesondere Methyl, ferner Ethyl, Propyl, Isopropyl, Butyl oder Isobutyl), Benzyl, o-, m-oder p-Methoxybenzyl, ferner o-, m- oder p-Methylbenzyl, 2,3-, 2,4-, 2,5-, 2,6-, 3,4- oder 3,5-Dimethylbenzyl, 2,3-, 2,4-, 2,5-, 2,6-, 3,4- oder 3,5-Dimethoxybenzyl, 3,4,5-Trimethoxybenzyl.

In R⁶ und R⁷ bedeuten vorzugsweise:
- A:: Methyl, ferner Ethyl;
- AO:: Methoxy, ferner Ethoxy;
- ACO:: Acetyl, ferner Propionyl;
- ACS:: Thioacetyl, ferner Thiopropionyl;
- AOOC: : Methoxycarbonyl, ferner Ethoxycarbonyl;
- AO-CS:: Methoxy-thiocarbonyl, ferner Ethoxythiocarbonyl;
- ACOO:: Acetoxy, ferner Propionoxy;
- ACSO:: Thio(no)acetoxy, ferner Thio(no)propionoxy;
- Hydroxy-alkyl:: Hydroxymethyl oder 1- oder 2-Hydroxyethyl;
- Mercapto-alkyl:: Mercaptomethyl oder 1- oder 2-Mercaptoethyl;
- NHA:: Methylamino, ferner Ethylamino;
- NA₂:: Dimethylamino, ferner Diethylamino;
- ASO:: Methylsulfinyl, ferner Ethylsulfinyl;
- ASO₂:: Methylsulfonyl, ferner Ethylsulfonyl;
- AO-SO:: Methoxy-sulfinyl, ferner Ethoxy-sulfinyl;
- AO-SO₂:: Methoxy-sulfonyl, ferner Ethoxy-sulfonyl;
- Ac-NH:: Acetamido, ferner Formamido, Propionamido oder Benzamido;
- AO-CO-NH:: Methoxycarbonylamino, ferner Ethoxycarbonylamino;
- HANSO:: Methylaminosulfinyl, ferner Ethylaminosulfinyl
- A₂NSO:: Dimethylaminosulfinyl, ferner Diethylaminosulfinyl;
- HANSO₂:: Methylaminosulfonyl, ferner Ethylaminosulfonyl;
- A₂NSO₂:: Dimethylaminosulfonyl, ferner Diethylaminosulfonyl;
- HANCO:: N-Methylcarbamoyl, ferner N-Ethylcarbamoyl;
- A₂NOC:: N,N-Dimethylcarbamoyl, ferner N,N-Diethylcarbamoyl;
- HANCS:: N-Methyl-thiocarbamoyl, ferner N-Ethyl-thiocarbamoyl;
- A₂NCS:: N,N-Dimethyl-thiocarbamoyl, ferner N,N-Diethyl-thiocarbamoyl;
- ASONH:: Methylsulfinylamino, ferner Ethylsulfinylamino:
- ASO₂NH:: Methylsulfonylamino, ferner Ethylsulfonylamino;
- AOSONH:: Methoxysulfinylamino, ferner Ethoxysulfinylamino;
- AOSO₂NH:: Methoxysulfonylamino, ferner Ethoxysulfonylamino;
- ACO-alkyl:: 2-Oxopropyl, 2-Oxobutyl, 3-Oxobutyl, 3-Oxopentyl;
- Nitro-alkyl:: Nitromethyl, 1- oder 2-Nitroethyl;
- Cyan-alkyl:: Cyanmethyl, 1- oder 2-Cyanethyl;
- A-C(= NOH):: 1-Oximinoethyl, ferner 1-Oximinopropyl;
- A-C(= NNH₂):: 1-Hydrazonoethyl, ferner 1-Hydrazonopropyl.

Die Reste R⁶ und R⁷ stehen vorzugsweise in 6- und 7-Stellung des Chromansystems. Sie können jedoch auch in 5- und 6-, 5- und 7-, 5- und 8-, 6- und 8- sowie 7- und 8-Stellung stehen.

Von den Resten R⁶ und R⁷ ist vorzugsweise der eine H, während der andere von H verschieden ist. Dieser andere Rest steht vorzugsweise in 6-Stellung, aber auch in 5-, 7- oder 8-Stellung, und ist vorzugsweise CN oder NO₂, ferner bevorzugt CHO, ACO (insbesondere Acetyl), AOOC (insbesondere Methoxycarbonyl oder Ethoxycarbonyl), ACOO (insbesondere Acetoxy), weiterhin bevorzugt F, Cl, Br, J, CF₃, H₂NCO, H₂NCS oder NH₂.

Der Rest R⁸ ist vorzugsweise H, weiterhin bevorzugt Methyl oder Ethyl.

Die Reste R⁹ und R¹⁰ sind vorzugsweise H, weiterhin bevorzugt A, insbesondere Methyl, NO₂, Cl oder Br.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die nachstehenden Formeln Ia bis Ii ausgedrückt werden, die der Formel I entsprechen und worin die nicht näher bezeichneten Reste die bei der Formel I angegebene Bedeutung haben, worin jedoch
- in Ia: R¹ und R² jeweils A bedeuten;
- in Ib: R¹ und R² jeweils CH₃ bedeuten;
- in Ic: R¹ und R² zusammen Alkylen mit 3-6 C-Atomen bedeuten;
- in Id: R⁵ A, Benzyl oder Methoxybenzyl bedeutet;
- in Ie: R⁵ Methyl, Benzyl oder p-Methoxybenzyl bedeutet;
- in If: R⁵ Methyl oder Benzyl bedeutet;
- in Ig: R¹ und R² jeweils CH₃ oder zusammen -(CH₂)₄-oder -(CH₂)₅-,
R⁵ A, Benzyl oder Methoxybenzyl,
R⁸ H oder CH₃ und
R⁹ und
R¹⁰ jeweils H bedeuten;
- in Ih: R¹ und R² jeweils CH₃,
R⁵ A, Benzyl oder Methoxybenzyl,
R⁸ H oder CH₃ und
R⁹ und
R¹⁰ jeweils H bedeuten;
- in Ii: R¹ und R² jeweils CH₃; R⁵ H, Methyl, Benzyl oder p-Methoxybenzyl und
R⁸, R⁹ und R¹⁰ jeweils H bedeuten.

Weiterhin sind bevorzugt Verbindungen der Formeln I′ sowie Ia′ bis Ii′, die den Formeln I sowie Ia bis Ii entsprechen, worin jedoch jeweils zusätzlich R³ OH, und R⁴ H bedeuten, insbesondere solche Verbindungen der Formeln I′ sowie Ia′ bis Ii′, worin jeweils zusätzlich R³ OH und R⁴ H bedeuten.

Weiterhin sind bevorzugt Verbindungen der Formeln I˝ sowie Ia˝ bis Ii˝, die den Formeln I sowie Ia bis Ii entsprechen, worin jedoch jeweils zusätzlich R³ und R⁴ zusammen eine Bindung bedeuten.

Ferner sind bevorzugt Verbindungen der Formeln I, I′, I˝, Ia bis Ii, Ia′ bis Ii′ sowie Ia˝ bis Ii˝, worin jeweils zusätzlich
(a) R⁶ von H verschieden ist und
   R⁷ H bedeutet;
(b) R⁶ von H verschieden ist und in 6-Stellung steht und
   R⁷ H bedeutet;
(c) R⁶ NO₂, CN, CHO, ACO, HOOC, AOOC, ACOO, F, Cl, Br, J, CF₃, H₂NCO, H₂NCS oder NH₂ und
R⁷ H bedeutet;
(d) R⁶ NO₂, CN, CHO, ACO, HOOC, AOOC, ACOO, F, Cl, Br, J, CF₃, H₂NCO, H₂NCS oder NH₂ bedeutet und in 6-Stellung steht und
   R⁷ H bedeutet;
(e) R⁶ NO₂, CN, CHO, CH₃CO, CH₃OOC,C₂H₅OOC oder CH₃COO und
   R⁷ H bedeutet;
(f) R⁶ NO₂, CN, CHO, CH₃CO, CH₃OOC,C₂H₅OOC oder CH₃COO bedeutet und in 6-Stellung steht und
   R⁷ H bedeutet;
(g) R⁶ NO₂ oder CN und
   R⁷ H bedeutet;
(h) R⁶ NO₂ oder CN bedeutet und in 6-Stellung steht und
   R⁷ H bedeutet;
(i) R⁶ CN und
   R⁷ H bedeutet;
(j) R⁶ CN bedeutet und in 6-Stellung steht und
   R⁷ H bedeutet.

Im übrigen haben vor- und nachstehend die Reste R¹ bis R¹⁰, A, "-alkyl" und Ac die bei Formel I angegebenen Bedeutungen, wenn nicht ausdrücklich etwas anderes angegeben ist.

Gegenstand der Erfindung ist ferner ein Verfahren zur Herstellung von Chromanderivaten der Formel I gemäß Anspruch 1, dadurch gekennzeichnet, daß man zur Herstellung einer Verbindung der Formel I, worin R⁵ von H verschieden ist, ein Chroman der Formel II 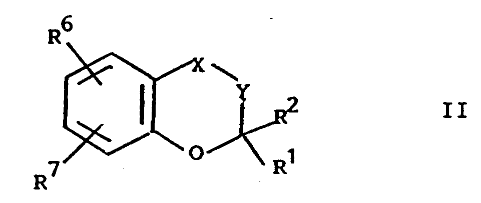
worin
- X-Y: 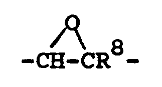 oder -CHE-CR³R⁸- und
- E: Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III 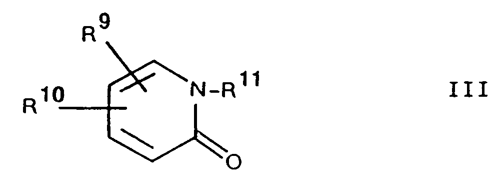
worin
- R¹¹: A oder einen unsubstituierten oder einen ein-, zwei- oder dreifach durch A und/oder OA substituierten Benzylrest bedeutet und
- R⁹ und R¹⁰: die bei der Formel I angegebenen Bedeutungen haben,
umsetzt,
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶, R⁷, R⁹ und/oder R¹⁰ in andere Reste R³, R⁵, R⁶, R⁷, R⁹ und/oder R¹⁰ umwandelt und/oder daß man eine basische Verbindung der Formel I durch Behandeln mit einer Säure in eines ihrer Säureadditionssalze umwandelt.

Die Verbindungen der Formel I werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart; Organic Reactions, John Wiley & Sons, Inc., New York; sowie in den oben angegebenen Patentanmeldungen) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Vorzugsweise werden diejenigen Verbindungen der Formel I, in denen R⁵ von H verschieden ist, durch Reaktion von Verbindungen der Formel II mit Verbindungen der Formel III hergestellt, zweckmäßig in Gegenwart eines inerten Lösungsmittels bei Temperaturen zwischen etwa 0 und 150 °.

Ausgangsstoffe der Formel II mit 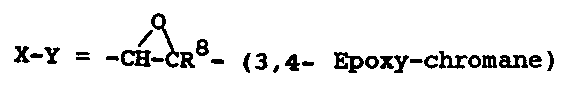
sind bevorzugt.

Die Ausgangsstoffe II und III sind in der Regel bekannt (vgl. z.B. DE-OS 37 26 261). Sofern sie nicht bekannt sind, können sie nach an sich bekannten Methoden hergestellt werden. So sind die Ausgangsstoffe der Formel II 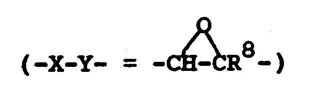
erhältlich durch Umsetzung von 2-Hydroxy-acetophenonen der Formel 2-HO-R⁶R⁷C₆H₂-COCH₃ mit Ketonen der Formel R¹-CO-R² zu entsprechenden 4-Chromanonen der Formel IVa

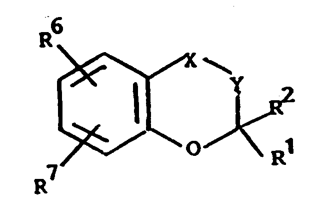

IVa -X-Y- = -CO-CH₂-

IVb -X-Y- = -CO-C(=CH-R¹²)-

IVc -X-Y- = -CHOH-CHR⁸-

IVd -X-Y- = -CH=CR⁸-

IVe -X-Y- = -CHBr-CR⁸OH-

gegebenenfalls Kondensation mit Aldehyden der Formel R¹²-CHO (R¹² = Alkyl mit 1-5 C-Atomen) zu 3-Alkyliden-4-chromanonen der Formel IVb, Reduktion z.B. mit NaBH₄ zu Chromanolen der Formel IVc, Dehydratisierung z.B. mit p-Toluolsulfonsäure, zu Chromenen der Formel IVd und Oxydation, z.B. mit 3-Chlorperbenzoesäure. Die letztgenannte Oxydation kann auch mehrstufig erfolgen. So kann man z.B. mit N-Bromsuccinimid in wäßriger Lösung zunächst die Bromhydrine der Formel IVe herstellen und aus diesen anschließend mit einer Base, z.B. Natronlauge, HBr abspalten.

Man kann die Chromene der Formel IVd auch erhalten durch Kondensation von Salicylaldehyden der Formel 2-HO-R⁶R⁷C₆H₂-CHO mit Ketonen der Formel R¹-CO-CH₂-R⁸ zu Hydroxyketonen der Formel 2-HO-R⁶R⁷C₆H₂-CH=CR⁸-CO-R¹, Umsetzung mit Organo-Li-Verbindungen der Formel R²-Li und nachfolgende Hydrolyse zu Diolen der Formel 2-HO-R⁶R⁷C₆H₂-CH=CR⁸-CR¹R²-OH und Cyclisierung unter Wasserabspaltung.

In Verbindungen der Formel II (-X-Y- = -CHE-CR³R⁸-) kommen als "reaktionsfähig veresterte OH-Gruppen" insbesondere die Ester mit Alkylsulfonsäuren (worin die Alkylgruppe 1-6 C-Atome enthält) oder mit Arylsulfonsäuren (worin die Arylgruppe 6-10 C-Atome enthält) in Betracht. Diese Verbindungen sind erhältlich aus den 4-Chromanolen der Formel IVc durch Umsetzung mit einem anorganischen Säurehalogenid wie PCl₃, PBr₃, SOCl₂ oder SOBr₂ oder mit einem Sulfonsäurechlorid wie Methan- oder p-Toluolsulfonsäurechlorid.

Bei der Umsetzung von II mit III ist es zweckmäßig, in Gegenwart einer Base zu arbeiten. Als Basen eignen sich bevorzugt Alkalimetall-alkyle, insbesondere Methyllithium oder Butyllithium, Alkalimetall-dialkylamide, insbesondere Lithiumdiisopropylamid, weiterhin z.B. Alkalimetall- oder Erdalkalimetall-hydroxide, -carbonate, -alkoholate, -hydride oder auch -amide wie NaOH, KOH, Ca(OH)₂, Na₂CO₃, K₂CO₃, Na- oder K-methylat, -ethylat oder -tert.-butylat, NaH, KH, CaH₂, NaNH₂, KNH₂, ferner organische Basen wie Triethylamin oder Pyridin, die auch im Überschuß angewendet werden können und dann gleichzeitig als Lösungsmittel dienen.

Als inerte Lösungmittel eignen sich insbesondere Alkohole wie Methanol, Ethanol, Isopropanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Nitrile wie Acetonitril; Nitroverbindungen wie Nitromethan oder Nitrobenzol; Ester wie Ethylacetat; Amide wie Dimethylformamid (DMF), Dimethylacetamid oder Phosphorsäurehexamethyltriamid; Sulfoxide wie Dimethylsulfoxid (DMSO); chlorierte Kohlenwasserstoffe wie Dichlormethan, Chloroform, Trichlorethylen, 1,2-Dichlorethan oder Kohlenstofftetrachlorid; Kohlenwasserstoffe wie Benzol, Toluol oder Xylol. Weiterhin eignen sich Gemische dieser Lösungsmittel untereinander.

Das Epoxid II 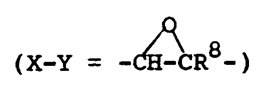
kann auch in situ hergestellt werden, z.B. durch Einwirkung einer Base auf das entsprechende Bromhydrin IVe.

Eine besonders bevorzugte Arbeitsweise besteht darin, daß man zunächst Diisopropylamin mit Butyllithium in THF/Hexan zu Lithiumdiisopropylamid umsetzt, dieses in situ mit dem Pyridon III zur Reaktion bringt, das Epoxid II hinzufügt und schließlich etwa 0,5 bis 20 Std. kocht.

Verbindungen der Formel I, worin R⁵ = H ist, werden zweckmäßig hergestellt, indem man eine sonst der Formel I entsprechende Verbindung, die jedoch an Stelle von R⁵ eine hydrogenolytisch oder solvolytisch abspaltbare Schutzgruppe R¹³ trägt, mit einem hydrogenolysierenden oder solvolysierenden Mittel behandelt.

Die Ausgangsstoffe sind z.B. zugänglich durch Reaktion eines Pyridons der Formel V 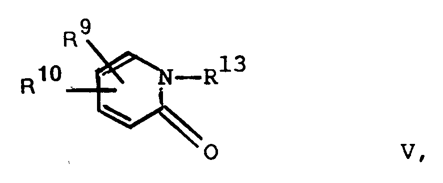
worin R⁹, R¹⁰ und R¹³ die angegebenen Bedeutungen haben, mit einem Epoxid der Formel II.

Als Schutzgruppen R¹³ kommen die üblichen Aminoschutzgruppen in Frage, wie sie z.B. in der Peptidchemie verwendet werden. Einige charakteristische Gruppen für R¹³ sind 2-Alkoxy-ethoxy-methyl wie 2-Methoxy-ethoxy-methyl ("MEM", abspaltbar z.B. mit ZnBr₂ oder TiCl₄ in Dichlormethan) oder 2-Trialkylsilyl-ethoxy-methyl wie 2-Trimethylsilyl-ethoxy-methyl ("SEM", abspaltbar z.B. mit F-Ionen).

Eine Verbindung der Formel I, worin R³ = OH und R⁴ = H ist, kann durch Behandeln mit einem Dehydratisierungsmittel in eine Verbindung der Formel I, worin R³ und R⁴ zusammen eine Bindung bedeuten, umgewandelt werden. Das gelingt z.B. durch Einwirkung einer der angegebenen Basen, z.B. NaOH oder NaH, in einem der angegebenen Lösungsmittel, z.B. THF, Dioxan oder DMSO, bei Temperaturen zwischen 0 und 150°.

Weiterhin kann man in einer Verbindung der Formel I einen oder mehrere der Reste R³, R⁵, R⁶, R⁷, R⁹ und/oder R¹⁰ in andere Reste R³, R⁵, R⁶, R⁷, R⁹ und/oder R¹⁰ umwandeln.

Beispielsweise ist es möglich, daß man ein H-Atom mittels einer Halogenierung durch ein Halogenatom oder mittels einer Nitrierung durch eine Nitrogruppe ersetzt und/oder eine Nitrogruppe zu einer Aminogruppe reduziert und/oder eine Amino- oder Hydroxygruppe alkyliert oder acyliert und/oder eine Cyangruppe (z.B. mit HCl in Wasser/Methanol bei 20-100°) in eine Carboxylgruppe oder (z.B. mit Raney-Nickel in Wasser/Essigsäure/Pyridin in Gegenwart von Natriumphosphat) in eine Formylgruppe oder (z.B. mit KOH in tert.-Butanol) in eine Carbamoylgruppe oder (z.B. mit H₂S in Pyridin/Triethylamin) in eine Thiocarbamoylgruppe umwandelt und/oder eine Carbamoylgruppe (z.B. mit POCl₃) zu einer Cyangruppe dehydratisiert und/oder eine -CO-NH-Gruppe (z.B. mit P₂S₅ oder mit Lawesson-Reagenz in Toluol) in eine -CS-NH- bzw. -C(SH)=N-Gruppe umwandelt und/oder eine Gruppe R⁵ = unsubstituierter oder wie angegeben substituierter Benzylrest hydrogenolytisch (z.B. mit H₂ an einem Katalysator wie Pd oder mit Ammoniumformiat in Methanol) abspaltet, wobei gleichzeitig ein 3-Chromen zum entsprechenden Chroman hydriert werden und/oder Cl-oder Br-Atome hydrogenolytisch abgespalten werden können.

Eine Nitrierung gelingt unter üblichen Bedingungen, z.B. mit einem Gemisch aus konzentrierter HNO₃ und konzentrierter H₂SO₄ bei Temperaturen zwischen 0 und 30°. Falls mindestens einer der Substituenten R⁶ und R⁷ eine elektronegative Gruppe wie CN oder NO₂ bedeutet, erfolgt die Nitrierung überwiegend am Pyridonring; andernfalls erhält man in der Regel Gemische, bei denen die Nitrogruppen am Pyridonring oder am Chromanring stehen können.

Analoges gilt für die Halogenierung, die z.B. mit elementarem Chlor oder Brom in einem der üblichen inerten Lösungsmittel bei Temperaturen zwischen etwa 0 und 30 ° durchgeführt werden kann.

Eine primäre oder sekundäre Aminogruppe und/oder eine OH-Gruppe kann durch Behandeln mit alkylierenden Mitteln in die entsprechende sekundäre oder tertiäre Aminogruppe und/oder Alkoxygruppe umgewandelt werden. Als alkylierende Mittel eignen sich z.B. Verbindungen der Formeln A-Cl, A-Br oder A-J oder entsprechende Schwefelsäure- oder Sulfonsäureester wie Methylchlorid, -bromid, -jodid, Dimethylsulfat, Methyl-p-toluolsulfonat. Ferner kann man z.B. eine oder zwei Methylgruppen mit Formaldehyd in Gegenwart von Ameisensäure einführen. Die Alkylierung wird zweckmäßig in Gegenwart oder Abwesenheit eines der genannten inerten Lösungemittel, z.B. DMF, bei Temperaturen zwischen etwa 0 ° und etwa 120 ° vorgenommen, wobei auch ein Katalysator zugegen sein kann, vorzugsweise eine Base wie Kalium-tert.-butylat oder NaH.

Als acylierende Mittel zur Acylierung von Amino- oder Hydroxygruppen eignen sich zweckmäßig die Halogenide (z.B. Chloride oder Bromide) oder Anhydride von Carbonsäuren der Formel Ac-OH, z.B. Acetanhydrid, Propionylchlorid, Isobutyrylbromid, Ameisensäure/Essigsäureanhydrid, Benzoylchlorid. Der Zusatz einer Base wie Pyridin oder Triethylamin bei der Acylierung ist möglich. Man acyliert zweckmäßig in Gegenwart oder Abwesenheit eines inerten Lösungsmittels, z.B. eines Kohlenwasserstoffs wie Toluol, eines Nitril wie Acetonitril, eines Amids wie DMF oder eines Überschusses einer tertiären Base wie Pyridin oder Triethylamin bei Temperaturen zwischen etwa 0° und etwa 160°, vorzugsweise zwischen 20° und 120°. Eine Formylierung gelingt auch mit Ameisensäure in Gegenwart von Pyridin.

Eine Base der Formel I kann mit einer Säure in das zugehörige Säureadditionssalz übergeführt werden. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2- oder 3-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono-und -disulfonsäuren, Laurylschwefelsäure. Salze mit physiologisch nicht unbedenklichen Säuren, z.B. Pikrate, können zur Aufreinigung der Verbindungen der Formel I verwendet werden.

Die Verbindungen der Formel I können ein oder mehrere chirale Zentren besitzen. Sie können daher bei ihrer Herstellung als Racemate oder, falls optisch aktive Ausgangsstoffe verwendet werden, auch in optisch aktiver Form erhalten werden. Weisen die Verbindungen zwei oder mehr chirale Zentren auf, dann können sie bei der Synthese als Gemische von Racematen anfallen, aus denen man die einzelnen Racemate, beispielsweise durch Umkristallisieren aus inerten Lösungsmitteln, in reiner Form isolieren kann. So haben z.B. Verbindungen der Formel I, worin R¹ = R², R³ = OH und R⁴ = H ist, zwei chirale Zentren; bei der Herstellung durch Reaktion von II mit III entsteht jedoch ganz überwiegend nur ein Racemat mit trans-Stellung der 1-R⁵-1,2-dihydro-2-oxo-3-pyridyl-gruppe und des Substituenten R³ = OH. Erhaltene Racemate können, falls erwünscht, nach an sich bekannten Methoden mechanisch, chemisch oder biochemisch in ihre Enantiomeren getrennt werden. So können aus dem Racemat durch Umsetzung mit einem optisch aktiven Trennmittel Diastereomere gebildet werden. Als Trennmittel für basische Verbindungen der Formel I eignen sich z.B. optisch aktive Säuren, wie die D- und L-Formen von Weinsäure, Dibenzoylweinsäure, Diacetylweinsäure, Camphansäure, Camphersulfonsäuren, Mandelsäure, Äpfelsäure oder Milchsäure. Carbinole (I, R³ = OH) können ferner mit Hilfe chiraler Acylierungsreagenzien, z.B. D- oder L-α-Methylbenzylisocyanat, verestert und dann getrennt werden (vgl. EP-A1-120428). Die verschiedenen Formen der Diastereomeren können in an sich bekannter Weise, z.B. durch fraktionierte Kristallisation, getrennt, und die Enantiomeren der Formel I können in an sich bekannter Weise aus den Diastereomeren in Freiheit gesetzt werden. Enantiomerentrennungen gelingen ferner durch Chromatographie an optisch-aktiven Trägermaterialien.

Die Verbindungen der Formel I und ihre physiologisch unbedenklichen Salze können zur Herstellung pharmazeutischer Zubereitungen verwendet werden, insbesondere auf nicht-chemischem Wege. Hierbei können sie zusammen mit mindestens einem festen, flüssigen und/oder halbflüssigen Träger-oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend mindestens eine Verbindung der Formel I und/oder eines ihrer physiologisch unbedenklichen Salze.

Diese Zubereitungen können als Arzneimittel in der Human-oder Veterinärmedizin verwendet werden. Als Trägerstoffe kommen organische oder anorganische Substanzen in Frage, die sich für die enterale (z. B. orale), parenterale oder topische Applikation eignen und mit den neuen Verbindungen nicht reagieren, beispielsweise Wasser, pflanzliche Öle, Benzylalkohole, Polyethylenglykole, Glycerintriacetat, Gelatine, Kohlehydrate wie Lactose oder Stärke, Magnesiumstearat, Talk, Lanolin, Vaseline. Zur oralen Anwendung dienen insbesondere Tabletten, Dragees, Kapseln, Sirupe, Säfte oder Tropfen, zur rektalen Anwendung Suppositorien, zur parenteralen Anwendung Lösungen, vorzugsweise ölige oder wässerige Lösungen, ferner Suspensionen, Emulsionen oder Implantate, für die topische Anwendung Salben, Cremes, Pasten, Lotionen, Gele, Sprays, Schäume, Aerosole, Lösungen (z.B. Lösungen in Alkoholen wie Ethanol oder Isopropanol, Acetonitril, DMF, Dimethylacetamid, 1,2-Propandiol oder deren Gemischen untereinander und/oder mit Wasser) oder Puder. Die neuen Verbindungen können auch lyophilisiert und die erhaltenen Lyophilisate z. B. zur Herstellung von Injektionspräparaten verwendet werden. Insbesondere für die topische Anwendung kommen auch liposomale Zubereitungen in Betracht. Die angegebenen Zubereitungen können sterilisiert sein und/oder Hilfsstoffe wie Gleit-, Konservierungs-, Stabilisierungs-und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Die Verbindungen der Formel I und ihre physiologisch unbedenklichen Salze können an Menschen oder Tiere, insbesondere Säugetiere wie Affen, Hunde, Katzen, Ratten oder Mäuse verabreicht und bei der therapeutischen Behandlung des menschlichen oder tierischen Körpers sowie bei der Bekämpfung von Krankheiten verwendet werden, insbesondere bei der Therapie und/oder Prophylaxe von Störungen des cardiovasculären Systems, insbesondere dekompensierter Herzinsuffizienz, Angina pectoris, Arrhythmie, peripheren oder cerebralen Gefäßerkrankungen, sowie Krankheitszuständen, die mit Bluthochdruck verbunden sind, ferner von Erkrankungen, die mit Veränderungen der nicht-vaskulären Muskulatur verbunden sind, z.B. Asthma, Inkontinenz der Harnblase.

Dabei werden die erfindungsgemäßen Substanzen in der Regel in Analogie zu bekannten Antianginosa bzw. Blutdrucksenkern, z. B. Nicorandil oder Cromakalim verabreicht, vorzugsweise in Dosierungen zwischen etwa 0,01 und 5 mg, insbesondere zwischen 0,02 und 0,5 mg pro Dosierungseinheit. Die tägliche Dosierung liegt vorzugsweise zwischen etwa 0,0001 und 0,1, insbesondere zwischen 0,0003 und 0,01 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, dem allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Die Verbindungen der Formel I und ihre Salze eignen sich, insbesondere bei topischer Anwendung, ferner zur Behandlung der Alopecia areata. Hierfür werden insbesondere pharmazeutische Zubereitungen verwendet, die zur topischen Behandlung der Kopfhaut geeignet und die oben genannt sind. Sie enthalten etwa 0,005 bis 10, bevorzugt 0,5 bis 3 Gew.% mindestens einer Verbindung der Formel I und/oder mindestens eines ihrer Salze. Im übrigen können diese Verbindungen gegen Alopezie in Analogie zu den Angaben in der WO 88/00822 verwendet werden.

In den nachfolgenden Beispielen bedeutet "übliche Aufarbeitung":
Man gibt, falls erforderlich, Wasser hinzu, extrahiert mit einem organischen Lösungsmittel wie Ethylacetat, trennt ab, trocknet die organische Phase über Natriumsulfat, filtriert, dampft ein und reinigt durch Chromatographie und/oder Kristallisation.

Vor- und nachstehend sind alle Temperaturen in °C angegeben.

### Beispiel 1

Eine Lösung von 1,4 ml Diisopropylamin in 10 ml THF wird unter Rühren und Einleiten von N₂ bei 0° mit 6,7 ml einer 15%igen Lösung von Butyllithium in Hexan versetzt. Danach wird eine Lösung von 1,85 g N-Benzyl-2-pyridon in 10 ml THF bei 0° zugetroft. Nach 0,5 Std. Rühren tropft man bei 0° eine Lösung von 2 g 2,2-Dimethyl-3,4-epoxy-6-cyanchroman in 10 ml THF hinzu, kocht 2 Std. und zersetzt überschüssiges Butyllithium durch Zutropfen von Methanol. Nach üblicher Aufarbeitung erhält man 2,2-Dimethyl-4-(1-benzyl-1,2-dihydro-2-oxo-3-pyridyl)-6-cyan-3-chromanol, F. 120-122°.

Analog erhält man mit den entsprechenden Pyridonen:
2,2-Dimethyl-4-(1-benzyl-1,2-dihydro-2-oxo-3-pyridyl)-6-nitro-3-chromanol
2,2-Dimethyl-4-(1-benzyl-1,2-dihydro-2-oxo-3-pyridyl)-6-fluor-3-chromanol
2,2-Dimethyl-4-(1-benzyl-1,2-dihydro-2-oxo-3-pyridyl)-6-chlor-3-chromanol
2,2-Dimethyl-4-(1-benzyl-1,2-dihydro-2-oxo-3-pyridyl)-6-brom-3-chromanol, F. 192-193°
2,2-Dimethyl-4-(1-benzyl-1,2-dihydro-2-oxo-3-pyridyl)-6-methoxycarbonyl-3-chromanol
2,2-Dimethyl-4-(1-benzyl-1,2-dihydro-2-oxo-3-pyridyl)-6-ethoxycarbonyl-3-chromanol
2,2-Tetramethylen-4-(1-benzyl-1,2-dihydro-2-oxo-3-pyridyl)-6-cyan-3-chromanol
2,2-Pentamethylen-4-(1-benzyl-1,2-dihydro-2-oxo-3-pyridyl)-6-cyan-3-chromanol
2,2-Hexamethylen-4-(1-benzyl-1,2-dihydro-2-oxo-3-pyridyl)-6-cyan-3-chromanol
2,2-Dimethyl-4-(1-methyl-1,2-dihydro-2-oxo-3-pyridyl)-6-cyan-3-chromanol ("A"), F. 247-248,5°
2,2-Dimethyl-4-(1-methyl-1,2-dihydro-2-oxo-3-pyridyl)-6-nitro-3-chromanol
2,2-Dimethyl-4-(1-methyl-1,2-dihydro-2-oxo-3-pyridyl)-6-brom-3-chromanol
2,2-Dimethyl-4-(1-methyl-1,2-dihydro-2-oxo-3-pyridyl)-6-methoxycarbonyl-3-chromanol
2,2-Dimethyl-4-(1-ethyl-1,2-dihydro-2-oxo-3-pyridyl)-6-cyan-3-chromanol
2,2-Dimethyl-4-(1-ethyl-1,2-dihydro-2-oxo-3-pyridyl)-6-nitro-3-chromanol
2,2-Dimethyl-4-(1-ethyl-1,2-dihydro-2-oxo-3-pyridyl)-6-brom-3-chromanol
2,2-Dimethyl-4-(1-ethyl-1,2-dihydro-2-oxo-3-pyridyl)-6-methoxycarbonyl-3-chromanol
2,2-Dimethyl-4-(1-p-methoxybenzyl-1,2-dihydro-2-oxo-3-pyridyl)-6-cyan-3-chromanol, F. 120°
2,2-Dimethyl-4-(1-p-methoxybenzyl-1,2-dihydro-2-oxo-3-pyridyl)-6-nitro-3-chromanol
2,2-Dimethyl-4-(1-p-methoxybenzyl-1,2-dihydro-2-oxo-3-pyridyl)-6-brom-3-chromanol
2,2-Dimethyl-4-(1-p-methoxybenzyl-1,2-dihydro-2-oxo-3-pyridyl)-6-methoxycarbonyl-3-chromanol.

### Beispiel 2

Eine Lösung von 2,82 g 2,2-Dimethyl-4-brom-6-cyan-3-chromanol und 1,09 g 1-Methyl-1,2-dihydro-2-oxo-pyridin in 70 ml DMSO wird mit 1,2 g 80%igem NaH versetzt und 3 Tage bei 20° gerührt. Nach üblicher Aufarbeitung erhält man "A", F. 247-248,5°.

### Beispiel 3

Zu einer Lösung von 1 g 2,2-Dimethyl-4-(1-benzyl-1,2-dihydro-2-oxo-3-pyridyl)-6-cyan-3-chromanol in 40 ml Dioxan gibt man unter Rühren und Einleiten von N₂ 0,4 g NaH, kocht anschließend 10 Std., dampft ein und arbeitet wie üblich auf. Man erhalt 2,2-Dimethyl-4-(1-benzyl-1,2-dihydro-2-oxo-3-pyridyl)-6-cyan-3-chromen, F. 130-132°.

Analog erhält man aus den entsprechenden 3-Chromanolen:
2,2-Dimethyl-4-(1-benzyl-1,2-dihydro-2-oxo-3-pyridyl)-6-nitro-3-chromen
2,2-Dimethyl-4-(1-benzyl-1,2-dihydro-2-oxo-3-pyridyl)-6-fluor-3-chromen
2,2-Dimethyl-4-(1-benzyl-1,2-dihydro-2-oxo-3-pyridyl)-6-chlor-3-chromen
2,2-Dimethyl-4-(1-benzyl-1,2-dihydro-2-oxo-3-pyridyl)-6-brom-3-chromen
2,2-Dimethyl-4-(1-benzyl-1,2-dihydro-2-oxo-3-pyridyl)-6-methoxycarbonyl-3-chromen
2,2-Dimethyl-4-(1-benzyl-1,2-dihydro-2-oxo-3-pyridyl)-6-ethoxycarbonyl-3-chromen
2,2-Tetramethylen-4-(1-benzyl-1,2-dihydro-2-oxo-3-pyridyl)-6-cyan-3-chromen
2,2-Pentamethylen-4-(1-benzyl-1,2-dihydro-2-oxo-3-pyridyl)-6-cyan-3-chromen
2,2-Hexamethylen-4-(1-benzyl-1,2-dihydro-2-oxo-3-pyridyl)-6-cyan-3-chromen
2,2-Dimethyl-4-(1-methyl-1,2-dihydro-2-oxo-3-pyridyl)-6-cyan-3-chromen, F. 187-188°
2,2-Dimethyl-4-(1-methyl-1,2-dihydro-2-oxo-3-pyridyl)-6-nitro-3-chromen
2,2-Dimethyl-4-(1-methyl-1,2-dihydro-2-oxo-3-pyridyl)-6-brom-3-chromen
2,2-Dimethyl-4-(1-methyl-1,2-dihydro-2-oxo-3-pyridyl)-6-methoxycarbonyl-3-chromen
2,2-Dimethyl-4-(1-ethyl-1,2-dihydro-2-oxo-3-pyridyl)-6-cyan-3-chromen
2,2-Dimethyl-4-(1-ethyl-1,2-dihydro-2-oxo-3-pyridyl)-6-nitro-3-chromen
2,2-Dimethyl-4-(1-ethyl-1,2-dihydro-2-oxo-3-pyridyl)-6-brom-3-chromen
2,2-Dimethyl-4-(1-ethyl-1,2-dihydro-2-oxo-3-pyridyl)-6-methoxycarbonyl-3-chromen
2,2-Dimethyl-4-(1-p-methoxybenzyl-1,2-dihydro-2-oxo-3-pyridyl)-6-cyan-3-chromen
2,2-Dimethyl-4-(1-p-methoxybenzyl-1,2-dihydro-2-oxo-3-pyridyl)-6-nitro-3-chromen
2,2-Dimethyl-4-(1-p-methoxybenzyl-1,2-dihydro-2-oxo-3-pyridyl)-6-brom-3-chromen
2,2-Dimethyl-4-(1-p-methoxybenzyl-1,2-dihydro-2-oxo-3-pyridyl)-6-methoxycarbonyl-3-chromen
2,2-Dimethyl-4-(1,2-dihydro-2-oxo-3-pyridyl)-6-cyan-3-chromen
2,2-Dimethyl-4-(1,2-dihydro-2-oxo-3-pyridyl)-6-nitro-3-chromen
2,2-Dimethyl-4-(1,2-dihydro-2-oxo-3-pyridyl)-6-fluor-3-chromen
2,2-Dimethyl-4-(1,2-dihydro-2-oxo-3-pyridyl)-6-chlor-3-chromen
2,2-Dimethyl-4-(1,2-dihydro-2-oxo-3-pyridyl)-6-brom-3-chromen
2,2-Dimethyl-4-(1,2-dihydro-2-oxo-3-pyridyl)-6-methoxycarbonyl-3-chromen
2,2-Dimethyl-4-(1,2-dihydro-2-oxo-3-pyridyl)-6-ethoxycarbonyl-3-chromen.

### Beispiel 4

Ein Gemisch von 5,8 g 2,2-Dimethyl-4-(1-benzyl-1,2-dihydro-2-oxo-3-pyridyl)-6-cyan-3-chromanol, 11,6 g KOH und 90 ml tert.-Butanol wird 50 Min. gekocht, abkühlt und auf Eis gegossen. Nach üblicher Aufarbeitung erhält man 2,2-Dimethyl-4-(1-Benzyl-1,2-dihydro-2-oxo-3-pyridyl)-6-carbamoyl-3-chromen, F. 199-202°.

### Beispiel 5

Ein Gemisch von 2 g "A", 11,7 ml Ameisensäure und 3,3 ml Acetanhydrid wird 16 Std. bei 20° stehengelassen und anschließend 1 Std. auf 40° erwärmt. Nach Eindampfen und üblicher Aufarbeitung erhält man 2,2-Dimethyl-3-formyloxy-4-(1-methyl-1,2-dihydro-2-oxo-3-pyridyl)-6-cyan-chroman.

### Beispiel 6

Ein Gemisch von 1 g "A" und 5 ml Acetanhydrid wird 1 Std. gekocht. Man kühlt ab, arbeitet wie üblich auf und erhält 2,2-Dimethyl-3-acetoxy-4-(1-methyl-1,2-dihydro-2-oxo-3-pyridyl)-6-cyan-chroman.

### Beispiel 7

Eine Lösung von 1 g 2,2-Dimethyl-4-(1-methyl-1,2-dihydro-2-oxo-3-pyridyl)-6-nitro-3-chromanol in 25 ml Methanol wird bei 20° und 1 bar an 0,5 g 5%igem Pd-C bis zum Stillstand hydriert. Man filtriert, dampft ein und erhält 2,2-Dimethyl-4-(1-methyl-1,2-dihydro-2-oxo-3-pyridyl)-6-amino-3-chromanol.

### Beispiel 8

Man hydriert eine Lösung von 4 g 2,2-Dimethyl-4-(1-benzyl-1,2-dihydro-2-oxo-3-pyridyl)-6-carbamoyl-3-chromen in 200 ml Methanol an 4 g 20%ig. Pd-C bei 20° und 1 bar, filtriert, dampft ein und erhält 2,2-Dimethyl-4-(1,2-dihydro-2-oxo-3-pyridyl)-6-carbamoyl-chroman, F. 177-179°.

### Beispiel 9

Eine Lösung von 1 g 2,2-Dimethyl-4-(1-methyl-1,2-dihydro-2-oxo-3-pyridyl)-6-amino-3-chromanol in 15 ml Ameisensäure und 1 ml Pyridin wird 24 Std. gekocht ud eingedampft. Nach üblicher Aufarbeitung erhält man 2,2-Dimethyl-4-(1-methyl-1,2-dihydro-2-oxo-3-pyridyl)-6-formamido-3-chromanol.

### Beispiel 10

Ein Gemisch von 1 g 2,2-Dimethyl-4-(1-methyl-1,2-dihydro-2-oxo-3-pyridyl)-6-amino-3-chromanol, 10 ml Acetanhydrid und 10 ml Pyridin wird 24 Std. bei 20° stehengelassen. Man dampft ein, arbeitet wie üblich auf und erhält 2,2-Dimethyl-4-(1-methyl-1,2-dihydro-2-oxo-3-pyridyl)-6-acetamido-3-chromanol.

### Beispiel 11

In eine siedende Lösung von 1 g "A" in 50 ml Methanol und 2 ml Wasser wird 12 Std. unter Rühren HCl eingeleitet. Man läßt erkalten und über Nacht stehen. Die ausgefallene 2,2-Dimethyl-4-(1-methyl-1,2-dihydro-2-oxo-3-pyridyl)-3-chromanol-6-carbonsäure wird abfiltriert.

### Beispiel 12

Ein Gemisch von 2,98 g 2,2-Dimethyl-4-(1,2-dihydro-2-oxo-3-pyridyl)-6-carbamoyl-chroman, 2 ml POCl₃ und 200 ml 1,2-Dichlorethan wird 45 Min. gekocht. Nach dem Abkühlen und üblicher Aufarbeitung erhält man 2,2-Dimethyl-4-(1,2-dihydro-2-oxo-3-pyridyl)-6-cyan-chroman, F. 174-175,5°.

Analog erhält man aus 2,2-Dimethyl-4-(1-benzyl-1,2-dihydro2-oxo-3-pyridyl)-6-carbamoyl-3-chromen das 2,2-Dimethyl-4-(1-benzyl-1,2-dihydro-2-oxo-3-pyridyl)-6-cyan-3-chromen.

### Beispiel 13

Ein Gemisch von 1 g "A", 10 g Trinatriumphosphat-dodekahydrat, 9 ml Pyridin, 9 ml Wasser, 22 ml Essigsäure und 8 g Raney-Nickel (wasserfeucht) wird bei 20° 3 Std. gerührt. Man filtriert, arbeitet wie üblich auf und erhält 2,2-Dimethyl-4-(1-methyl-1,2-dihydro-2-oxo-3-pyridyl)-6-formyl-3-chromanol.

### Beispiel 14

In eine Lösung von 1 g "A" in einem Gemisch von 7 ml Pyridin und 7 ml Triethylamin leitet man 3 Std. bei 20° H₂S ein, dampft ein, arbeit wie üblich auf und erhält 2,2-Dimethyl-4-(1-methyl-1,2-dihydro-2-oxo-3-pyridyl)-6-thiocarbamoyl-3-chromanol.

### Beispiel 15

Ein Gemisch von 386 mg 2,2-Dimethyl-4-(1-benzyl-1,2-dihydro-2-oxo-3-pyridyl)-6-cyan-3-chromanol, 400 mg 10%ig. Pd-C, 315 mg Ammoniumformiat und 7 ml Methanol wird unter N₂ 20 Min. gekocht. Man kühlt ab, filtriert, arbeit wie üblich auf und erhält 2,2-Dimethyl-4-(1,2-dihydro-2-oxo-3-pyridyl)-6-cyan-3-chromanol.

### Beispiel 16

Man hydriert eine Lösung von 280 mg 2,2-Dimethyl-4-(1-benzyl-1,2-dihydro-2-oxo-3-pyridyl)-6-brom-3-chromanol in 80 ml Methanol an 140 mg 20%ig. Pd-C bei 20° und 1 bar, filtriert, dampft ein und erhält nach chromatographischer Reinigung an Kieselgel 2,2-Dimethyl-4-(1,2-dihydro-2-oxo-3-pyridyl)-3-chromanol, F. 244-246°.

### Beispiel 17

Ein Gemisch aus 1 g 2,2-Dimethyl-4-(1-benzyl-1,2-dihydro-2-oxo-3-pyridyl)-6-cyan-3-chromanol und 10 ml 85%iger Phosphorsäure wird 2 Stunden auf 100° erwärmt. Nach dem Abkühlen und üblicher Aufarbeitung erhält man 2,2-Dimethyl-4-(1-benzyl-1,2-dihydro-2-oxo-3-pyridyl)-6-carbamoyl 3-chromanol,F. 230-235°.

### Beispiel 18

Analog Beispiel 9 erhält man aus 2,2-Dimethyl-4-(1-benzyl-1,2-dihydro-2-oxo-3-pyridyl)-6-carbamoyl-3-chromanol mit H₂ an 20%ig. Pd-C in Methanol das 2,2-Dimethyl-4-(1,2-dihydro-2-oxo-3-pyridyl)-6-carbamoyl-3-chromanol, kein F. bis 280°.

### Beispiel 19

Analog Beispiel 4 erhält man aus 2,2-Dimethyl-4-(1-benzyl-1,2-dihydro-2-oxo-3-pyridyl)-6-brom-3-chromanol und NaH in Dioxan das 2,2-Dimethyl-4-(1-benzyl-1,2-dihydro-2-oxo-3-pyridyl)-6-brom-3-chromen (F. 118-119°),das analog Beispiel 9 zu 2,2-Dimethyl-4-(1,2-dihydro-2-oxo-3-pyridyl)-chroman hydriert wird; F. 185-186°.

Die nachstehenden Beispiele betreffen pharmazeutische Zubereitungen, welche Verbindungen der Formel I und/oder deren physiologisch unbedenkliche Salze enthalten.

### Beispiel A: Tabletten

Ein Gemisch von 0,2 kg "A", 136,3 kg Calciumhydrogenphosphat, 15 kg Maisstärke, 10 kg mikrokristalliner Cellulose, 5,5 kg unlöslichem Polyvinylpyrrolidon (PVP), 1,5 kg hochdispersem Siliciumdioxid und 1,5 kg Magnesiumstearat wird in üblicher Weise zu Tabletten gepreßt. Jede 170-mg-Tablette enthält 0,2 mg Wirkstoff.

### Beispiel B: Dragees

Analog Beispiel A, jedoch ohne den Zusatz von PVP, werden Tabletten gepreßt, die anschließend in üblicher Weise mit einem Überzug aus Saccharose, Maisstärke, Talk, Tragant und Farbstoff überzogen werden.

### Beispiel C: Lacktabletten

Aus 0,2 kg 2,2-Dimethyl-4-(1-benzyl-1,2-dihydro-2-oxo-3-pyridyl)-6-cyan-3-chromanol, 151,3 kg Lactose, 10 kg mikrokristalliner Cellulose, 5,5 kg unlöslichem PVP, 1,5 kg hochdispersem Siliciumdioxid und 1,5 kg Calciumstearat werden Tablettenkerne (170 mg) gepreßt, die anschließend in üblicher Weise lackiert werden, so daß jede Lacktablette mit 3,922 mg eines Lackes überzogen ist, der aus 2,2 mg Hydroxypropylmethylcellulose, 0,53 mg Polyethylenglykol 400, 0,85 mg Titandioxid, 0,12 mg Eisen(III)-oxid (gelb), 0,002 mg Eisen(III)-oxid (rot) und 0,22 mg Silikonöl besteht.

### Beispiel D : Kapseln

Man bereitet ein Granulat aus 10 g 2,2-Dimethyl-4-(1-methyl-1,2-dihydro-2-oxo-3-pyridyl)-6-cyan-3-chromen, 27,5 kg Lactose, 0,35 kg Hydroxypropylmethylcellulose und 0,7 kg Maisstärke, mischt dieses mit 0,15 kg hochdispersem Siliciumdioxid und 0,3 kg Magnesiumstearat und füllt das Gemisch in üblicher Weise in Hartgelatinekapseln, so daß jede Kapsel 0,1 mg Wirkstoff enthält.

### Beispiel E: Ampullen

Eine Lösung von 10 g "A" in 70 l 1,2-Propandiol wird mit zweifach destilliertem Wasser auf 100 l aufgefüllt, steril filtriert, und in 1 ml-Ampullen abgefüllt, die dann steril verschlossen werden. Jede Ampulle enthält 0,1 mg Wirkstoff.

## Patentansprüche (Patentansprüche für folgende(n) Vertragsstaat(en): AT, BE, CH, LI, DE, DK, FR, GB, GR, IT, LU, NL, SE)

1. Chromanderivate der Formel I 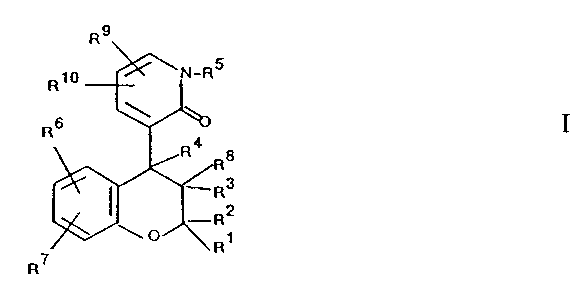 worin
R¹ und R² jeweils A,
R⁸ H,
R³ OH,
R⁴ H
R³ und R⁴ zusammen auch eine Bindung,
R⁵ A oder einen unsubstituierten oder einfach durch A oder OA substituierten Benzylrest,
R⁶ und R⁷ jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
R⁹ und R¹⁰ jeweils H, A, F, Cl, Br, J, OH, OA, OAc, SH, NO₂, NH₂, NHAc, COOH oder COOA,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze.

2. a) 2,2-Dimethyl-4-(1-benzyl-1,2-dihydro-2-oxo-3-pyridyl)-6-cyan-3-chromanol;
b) 2,2-Dimethyl-4-(1-methyl-1,2-dihydro-2-oxo-3-pyridyl)-6-cyan-3-chromanol.

3. Verfahren zur Herstellung von Chromanderivaten der Formel I gemäß Anspruch 1, dadurch gekennzeichnet, daß man zur Herstellung einer Verbindung der Formel I, worin R⁵ von H verschieden ist, ein Chroman der Formel II 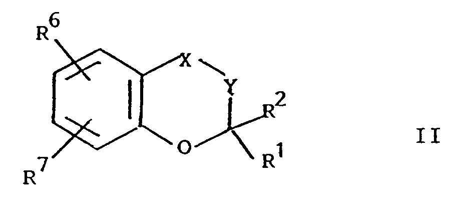 worin
X-Y 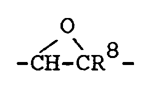 oder -CHE-CR³R⁸- und
E Cl, Br, J oder eine reaktionsfähig veresterte OH-Gruppe bedeuten und
R¹, R², R³ R⁶ R⁷ und R⁸ die bei Formel I angegebenen Bedeutungen haben,
mit einer Verbindung der Formel III 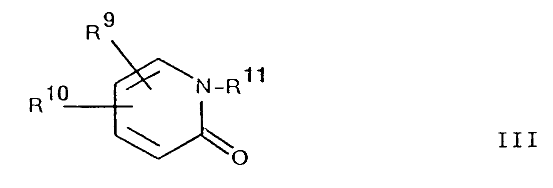 worin
R¹¹ A oder einen unsubstituierten oder einen einfach durch A oder OA substituierten Benzylrest bedeutet und
R⁹ und R¹⁰ die bei der Formel I angegebenen Bedeutungen haben,
umsetzt, und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶, R⁷, R⁹ und/oder R¹⁰ in andere Reste R³, R⁵, R⁶, R⁷, R⁹ und/oder R¹⁰ umwandelt und/oder daß man eine basische Verbindung der Formel I durch Behandeln mit einer Saure in eines ihrer Säureadditionssalze umwandelt.

4. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verwendung von Verbindungen der Formel I zur Herstellung eines Arzneimittels.

## Patentansprüche (Patentansprüche für folgende(n) Vertragsstaat(en): ES)

1. Verfahren zur Herstellung von Chromanderivaten der Formel I 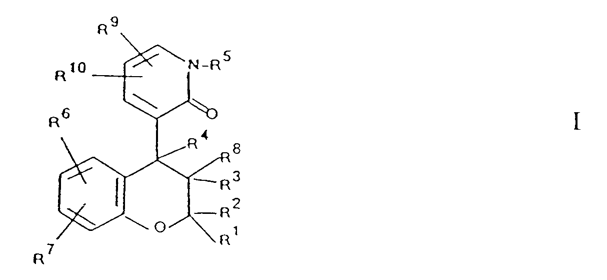 worin
R¹ und R² jeweils A,
R⁸ H,
R³ OH,
R⁴ H
R³ und R⁴ zusammen auch eine Bindung,
R⁵ A oder einen unsubstituierten oder einfach durch A oder OA substituierten Benzylrest,
R⁶ und R⁷ jeweils H, A, HO, AO, CHO, ACO, ACS, HOOC, AOOC, AO-CS, ACOO, A-CS-O, Hydroxy-alkyl, Mercapto-alkyl, NO₂, NH₂, NHA, NA₂, CN, F, Cl, Br, J, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, Nitro-alkyl, Cyan-alkyl, A-C(=NOH) oder A-C(=NNH₂),
R⁹ und R¹⁰ jeweils H, A, F, Cl, Br, J, OH, OA, OAc, SH, NO₂, NH₂, NHAc, COOH oder COOA,
A Alkyl mit 1-6 C-Atomen,
-alkyl Alkylen mit 1-6 C-Atomen und
Ac Alkanoyl mit 1-8 C-Atomen oder Aroyl mit 7-11 C-Atomen
bedeuten,
sowie deren Salze, dadurch gekennzeichnet,
daß man zur Herstellung einer Verbindung der Formel I, worin R⁵ von H verschieden ist, ein Chroman der Formel II 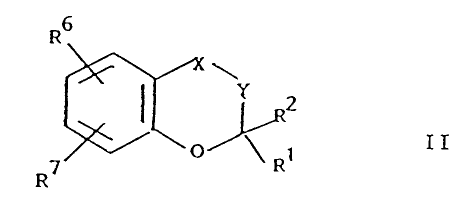 worin
X-Y 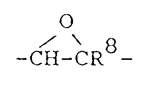 oder -CHE-CR³R⁸- und
E Cl, Br, J oder eine reaktionsfähig veresterte OH-Gruppe bedeuten und
R¹, R², R³, R⁶, R⁷ und R⁸ die bei Formel I angegebenen Bedeutungen haben,
mit einer Verbindung der Formel III 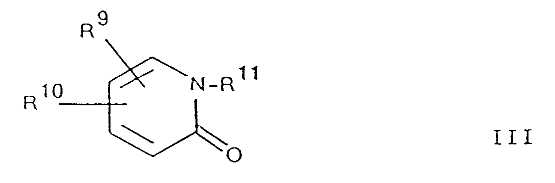 worin
R¹¹ A oder einen unsubstituierten oder einen einfach durch A oder OA substituierten Benzylrest bedeutet und
R⁹ und R¹⁰ die bei der Formel I angegebenen Bedeutungen haben,
umsetzt,
und/oder daß man eine Verbindung der Formel I, worin R³ OH und R⁴ H bedeutet, dehydratisiert und/oder daß man in einer Verbindung der Formel I einen oder mehrere der Reste R³, R⁵, R⁶, R⁷, R⁹ und/oder R¹⁰ in andere Reste R³, R⁵, R⁶, R⁷, R⁹ und/oder R¹⁰ umwandelt und/oder daß man eine basische Verbindung der Formel I durch Behandeln mit einer Säure in eines ihrer Säureadditionssalze umwandelt.

2. Verfahren zur Herstellung von
a) 2,2-Dimethyl-4-(1-benzyl-1,2-dihydro-2-oxo-3-pyridyl)-6-cyan-3-chromanol;
b) 2,2-Dimethyl-4-(1-methyl-1,2-dihydro-2-oxo-3-pyridyl)-6-cyan-3-chromanol
gemäß Anspruch 1.

3. Verfahren zur Herstellung pharmazeutischer Zubereitungen, dadurch gekennzeichnet, daß man eine Verbindung der Formel I und/oder eines ihrer physiologisch unbedenklichen Salze zusammen mit mindestens einem festen, flüssigen oder halbflüssigen Träger- oder Hilfsstoff in eine geeignete Dosierungsform bringt.

## Claims (Claims for the following Contracting State(s): AT, BE, CH, LI, DE, DK, FR, GB, GR, IT, LU, NL, SE)

1. Chroman derivatives of the formula I 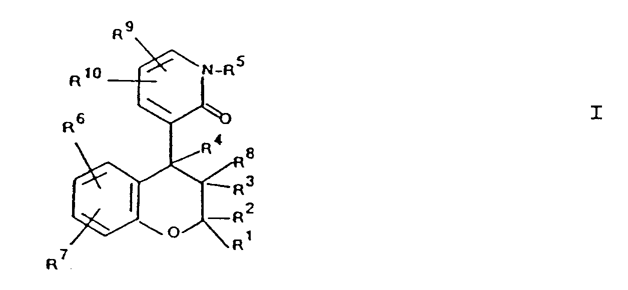 in which
R¹ and R² are each A,
R⁸ is H,
R³ is OH,
R⁴ is H,
R³ and R⁴ together are also a bond,
R⁵ is A or a benzyl radical which is unsubstituted, monosubstituted, disubstituted or trisubstituted by A or OA,
R⁶ and R⁷ are each H, A, HO, AO, CHO, ACO, ACS, HOOC, AOOC, AO-CS, ACOO, A-CS-O, hydroxyalkyl, mercaptoalkyl, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
R⁹ and R¹⁰ are each H, A, F, Cl, Br, I, OH, OA, OAC, SH, NO₂, NH₂, NHAc, COOH or COOA,
A is alkyl having 1-6 C atoms,
-alkyl is alkylene having 1-6 C atoms and
Ac is alkanoyl having 1-8 C atoms or aroyl having 7-11 C atoms
and their salts.

2. a) 2,2-Dimethyl-4-(1-benzyl-1,2-dihydro-2-oxo-3-pyridyl)-6-cyano-3-chromanol;
b) 2,2-Dimethyl-4-(1-methyl-1,2-dihydro-2-oxo-3-pyridyl)-6-cyano-3-chromanol.

3. Process for the preparation of chroman derivatives of the formula I according to Claim 1, characterized in that in order to prepare a compound of the formula I, in which R⁵ is different from H, a chroman of the formula II 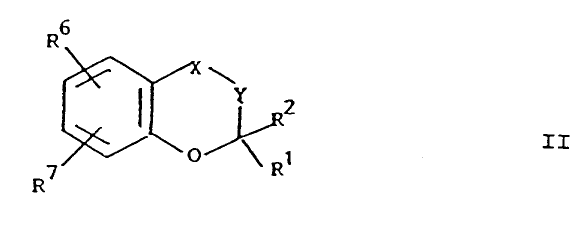 in which
X-Y is 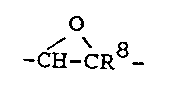 or -CHE-CR³R⁸- and
E is Cl, Br, I or a reactively esteriped OH group and R¹, R², R³, R⁶, R⁷ and R⁸ have the meanings given in formula I, is reacted with a compound of the formula III 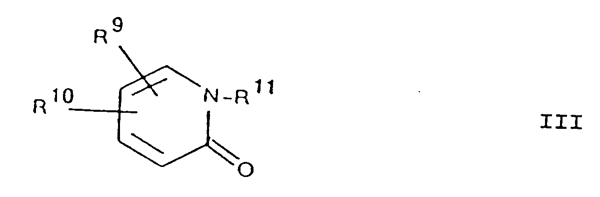 in which
R¹¹ is A or a benzyl radical which is unsubstituted or monosubstitued by A or OA and
R⁹ and R¹⁰ have the meanings indicated in the formula I,
and/or in that a compound of the formula I, in which R³ is OH and R⁴ is H, is dehydrated and/or in that in a compound of the formula I, one or more of the radicals R³, R⁵, R⁶, R⁷, R⁹ and/or R¹⁰ are converted into other radicals R³, R⁵, R⁶, R⁷, R⁹ and/or R¹⁰ and/or in that a basic compound of the formula I is converted into one of its acid addition salts by treating with an acid.

4. Process for the production of pharmaceutical preparations, characterized in that a compound of the formula I and/or one of its physiologically acceptable salts is brought into a suitable form for administration together with at least one solid, liquid or semi-liquid excipient or auxiliary.

5. Pharmaceutical preparation which contains at least one compound of the formula I and/or one of its physiologically acceptable salts.

6. Use of compounds of the formula I for the production of a medicament.

## Claims (Claims for the following Contracting State(s): ES)

1. Process for the preparation of chroman derivatives of the formula I 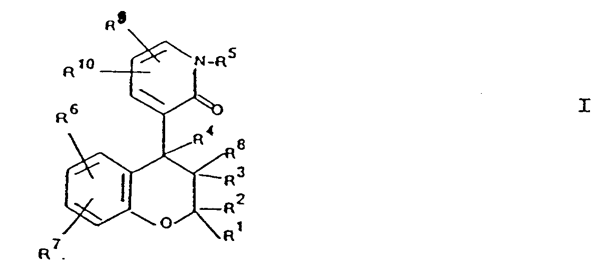 in which
R¹ and R² are each A,
R⁸ is H,
R³ is OH,
R⁴ is H,
R³ and R⁴ together are also a bond,
R⁵ is A or a benzyl radical which is unsubstituted, monosubstituted, disubstituted or trisubstituted by A or OA,
R⁶ and R⁷ are each H, A, HO, AO, CHO, ACO, ACS, HOOC, AOOC, AO-CS, ACOO, A-CS-O, hydroxyalkyl, mercaptoalkyl, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, ACO-alkyl, nitroalkyl, cyanoalkyl, A-C(=NOH) or A-C(=NNH₂),
R⁹ and R¹⁰ are each H, A, F, Cl, Br, I, OH, OA, OAC, SH, NO₂, NH₂, NHAc, COOH or COOA,
A is alkyl having 1-6 C atoms,
-alkyl is alkylene having 1-6 C atoms and
Ac is alkanoyl having 1-8 C atoms or aroyl having 7-11 C atoms
and their salts, characterized in that in order to prepare a compound of the formula I, in which R⁵ is different from H, a chroman of the formula II 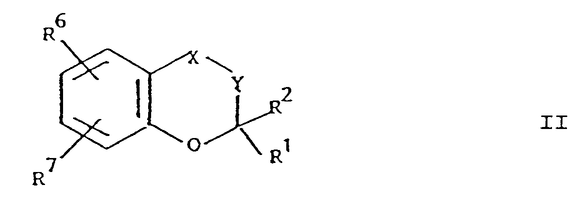 in which X-Y is 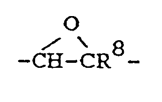
or -CHE-CR³R⁸- and
E is Cl, Br, I or a reactively esteriped OH group and R¹, R², R³, R⁶, R⁷ and R⁸ have the meanings given in formula I, is reacted with a compound of the formula III 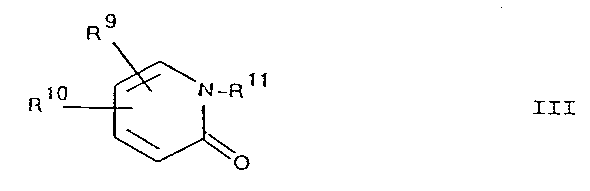 in which
R¹¹ is A or a benzyl radical which is unsubstituted or monosubstitued by A or OA and
R⁹ and R¹⁰ have the meanings indicated in the formula I,
and/or in that a compound of the formula I, in which R³ is OH and R⁴ is H, is dehydrated and/or in that in a compound of the formula I, one or more of the radicals R³, R⁵, R⁶, R⁷, R⁹ and/or R¹⁰ are converted into other radicals R³, R⁵, R⁶, R⁷, R⁹ and/or R¹⁰ and/or in that a basic compound of the formula I is converted into one of its acid addition salts by treating with an acid.

2. Process for the preparation of
a) 2,2-Dimethyl-4-(1-benzyl-1,2-dihydro-2-oxo-3-pyridyl)-6-cyano-3-chromanol;
b) 2,2-Dimethyl-4-(1-methyl-1,2-dihydro-2-oxo-3-pyridyl)-6-cyano-3-chromanol according to claim 1.

3. Process for the production of pharmaceutical preparations, characterized in that a compound of the formula I and/or one of its physiologically acceptable salts is brought into a suitable form for administration together with at least one solid, liquid or semi-liquid excipient or auxiliary.

## Revendications (Revendications pour l'(les) Etat(s) contractant(s) suivant(s): AT, BE, CH, LI, DE, DK, FR, GB, GR, IT, LU, NL, SE)

1. Dérivés de chromane répondant à la formule I 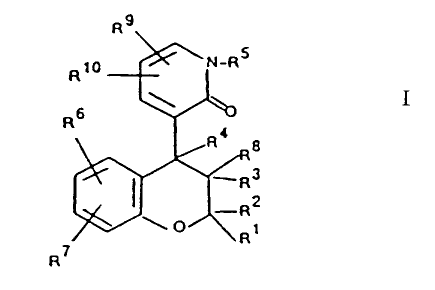 dans laquelle
R¹ et R² représentent chacun A,
R⁸ représente H,
R³ représente OH,
R⁴ représente H,
R³ et R⁴ représentent ensemble également une liaison,
R⁵ représente A ou encore un radical benzyle non substitué ou substitué une fois par A ou par OA,
R⁶ et R⁷ représentent chacun H, A, HO, AO, CHO, ACO, ACS, HOOC, AOOC, AO-CS, ACOO, A-CS-O, un groupe hydroxyalkyle, un groupe mercaptoalkyle, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, un groupe ACO-alkyle, un groupe nitro-alkyle, un groupe cyano-alkyle, A-C(=NOH) ou A-C(=NNH₂),
R⁹ et R¹⁰ représentent chacun H, A, F, Cl, Br, I, OH, OA, OAc, SH, NO₂, NH₂, NHAc, COOH ou COOA,
A représente un groupe alkyle contenant de 1 à 6 atomes de carbone,
-alkyle représente un groupe alkylène contenant de 1 à 6 atomes de carbone,
Ac représente un groupe alcanoyle contenant de 1 à 8 atomes de carbone ou un groupe aroyle contenant de 7 à 11 atomes de carbone,
ainsi que leurs sels.

2. a) 2,2-diméthyl-4-(1-benzyl-1,2-dihydro-2-oxo-3-pyridyl)-6-cyano-3-chromanol;
b) 2,2-diméthyl-4-(1-méthyl-1,2-dihydro-2-oxo-3-pyridyl)-6-cyano-3-chromanol.

3. Procédé pour la préparation de dérivés de chromane de formule I selon la revendication 1, caractérisé en ce que, pour la préparation d'un composé de formule I dans laquelle R⁵ est différent de H, on fait réagir un chromane répondant à la formule II 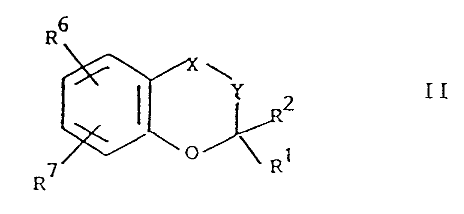 dans laquelle
X-Y représente 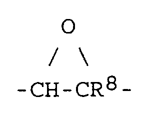 ou -CHE-CR³R⁸-, et
E représente Cl, Br, I ou un groupe OH estérifié apte à réagir, et
R¹, R², R³, R⁶, R⁷ et R⁸ ont les significations indiquées dans la formule I,
avec un composé de formule III 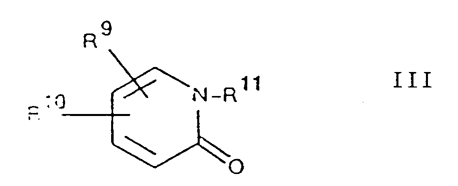 dans laquelle
R¹¹ représente A ou bien un radical benzyle non substitué ou encore substitué une fois par A ou par OA,
R⁹ et R¹⁰ ont les significations indiquées dans la formule I,
et/ou en ce qu'on soumet un composé de formule I dans laquelle R³ représente OH et R⁴ représente H, à une déshydratation et/ou en ce qu'on transforme, dans un composé de formule I, un ou plusieurs des radicaux R³, R⁵, R⁶, R⁷, R⁹ et/ou R¹⁰ en d'autres radicaux R³, R⁵, R⁶, R⁷, R⁹ et/ou R¹⁰, et/ou en ce qu'on transforme un composé basique de formule I par traitement avec un acide en l'un de ses sels d'addition d'acides.

4. Procédé pour la formulation de préparations pharmaceutiques, caractérisé en ce qu'on amène un composé de formule I et/ou un de ses sels physiologiquement acceptables conjointement avec au moins une substance de support ou une substance auxiliaire solide, liquide ou semi-liquide dans une forme posologique appropriée.

5. Préparation pharmaceutique caractérisée par une teneur en au moins un composé de formule I et/ou en un de ses sels physiologiquement acceptables.

6. Utilisation de composés de formule I pour la préparation d'un médicament.

## Revendications (Revendications pour l'(les) Etat(s) contractant(s) suivant(s): ES)

1. Procédé de préparation des dérivés du chromane répondant à la formule I 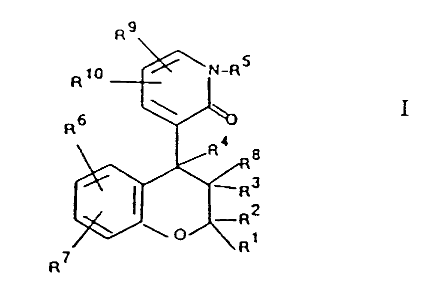 dans laquelle
R¹ et R² représentent chacun A,
R⁸ représente H,
R³ représente OH,
R⁴ représente H,
R³ et R⁴ représentent ensemble également une liaison,
R⁵ représente A ou encore un radical benzyle non substitué ou substitué une fois par A ou par OA,
R⁶ et R⁷ représentent chacun H, A, HO, AO, CHO, ACO, ACS, HOOC, AOOC, AO-CS, ACOO, A-CS-O, un groupe hydroxyalkyle, un groupe mercaptoalkyle, NO₂, NH₂, NHA, NA₂, CN, F, Cl, Br, I, CF₃, ASO, ASO₂, AO-SO, AO-SO₂, AcNH, AO-CO-NH, H₂NSO, HANSO, A₂NSO, H₂NSO₂, HANSO₂, A₂NSO₂, H₂NCO, HANCO, A₂NCO, H₂NCS, HANCS, A₂NCS, ASONH, ASO₂NH, AOSONH, AOSO₂NH, un groupe ACO-alkyle, un groupe nitro-alkyle, un groupe cyano-alkyle, A-C(=NOH) ou A-C(=NNH₂),
R⁹ et R¹⁰ représentent chacun H, A, F, Cl, Br, I, OH, OA, OAc, SH, NO₂, NH₂, NHAc, COOH ou COOA,
A représente un groupe alkyle contenant de 1 à 6 atomes de carbone,
-alkyle représente un groupe alkylène contenant de 1 à 6 atomes de carbone,
Ac représente un groupe alcanoyle contenant de 1 à 8 atomes de carbone ou un groupe aroyle contenant de 7 à 11 atomes de carbone,
ainsi que leurs sels, caractérisé en ce que,
pour la préparation d'un composé de formule I dans laquelle R⁵ est différent de H, on fait réagir un chromane répondant à la formule II 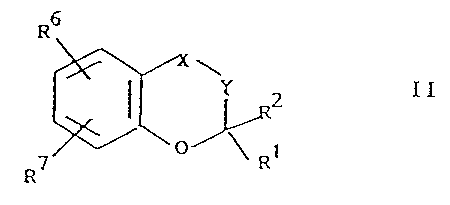 dans laquelle
X-Y représente 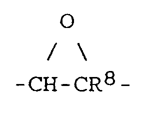 ou -CHE-CR³R⁸-, et
E représente Cl, Br, I ou un groupe OH estérifié apte à réagir, et
R¹, R², R³, R⁶, R⁷ et R⁸ ont les significations indiquées dans la formule I,
avec un composé de formule III 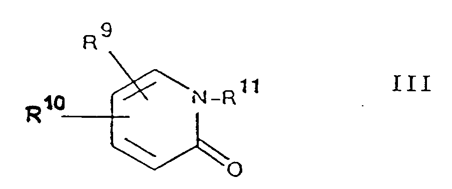 dans laquelle
R¹¹ représente A ou bien un radical benzyle non substitué ou encore substitué une fois par A ou par OA,
R⁹ et R¹⁰ ont les significations indiquées dans la formule I,
et/ou en ce qu'on soumet un composé de formule I dans laquelle R³ représente OH et R⁴ représente H, à une déshydratation et/ou en ce qu'on transforme, dans un composé de formule I, un ou plusieurs des radicaux R³, R⁵, R⁶, R⁷, R⁹ et/ou R¹⁰ en d'autres radicaux R³, R⁵, R⁶, R⁷, R⁹ et/ou R¹⁰, et/ou en ce qu'on transforme un composé basique de formule I par traitement avec un acide en l'un de ses sels d'addition d'acides.

2. Procédé de préparation de :
a) 2,2-diméthyl-4-(1-benzyl-1,2-dihydro-2-oxo-3-pyridyl)-6-cyano-3-chromanol;
b) 2,2-diméthyl-4-(1-méthyl-1,2-dihydro-2-oxo-3-pyridyl)-6-cyano-3-chromanol
selon revendication 1.

3. Procédé de préparation de compositions pharmaceutiques, caractérisé en ce que l'on met sous une la forme de dosage appropriée un composé de formule I et/ou l'un de ses sels acceptables pour l'usage pharmaceutique avec au moins un véhicule ou produit auxiliaire solide, liquide ou semi-liquide.
